# EUROPEAN PATENT APPLICATION

(11) **EP 3 563 756 A1**
(43) Date of publication of application: **06.11.2019**
(21) Application number: 18170001.4
(22) Date of filing: 30.04.2018
(51) Int. Cl.: A61B 3/12, A61F 9/007, A61B 5/00

(54) **APPARATUS AND METHOD FOR APPLYING PRESSURE PROXIMATE AN EYE**

(71) Applicant: Nokia Technologies Oy, 02610 Espoo (FI)
(72) Inventor: MULLER, Kiti Meerit Irmeli, 00430 Helsinki (FI); KÄRKKÄINEN, Leo, 00100 Helsinki (FI); HUTTUNEN, Janne, 02610 Espoo (FI); MURGAS, Matej, 00240 Helsinki (FI)
(74) Representative: Nokia EPO representatives

(57) **Abstract**

An example method and an example apparatus are provided to controllably induce venous pulsation, such as during an eye examination including inspection of the eye fundus. An example apparatus is provided that includes first means configured to sense an amount of pressure that is applied and transfer substantially the amount of pressure that is sensed to a location proximate the eye of a subject. The first means is configured to transfer substantially the amount of pressure that is sensed to a location proximate an eye of the subject without making direct contact with a surface of the eye. The apparatus also includes second means, which may be the first means, configured to transmit a signal indicative of substantially the amount of pressure that is sensed to a receiving device.

## Description

### TECHNICAL FIELD

The subject matter described herein relates generally to ophthalmoscopy and, more particularly, to a method and apparatus for applying pressure to or near an eye of a subject for various purposes.

### BACKGROUND

During a clinical examination, an examiner uses an ophthalmoscope to study the optical fundus in order to obtain information regarding the health and fitness of the subject including the health of the subject's eye. The fundus is the interior surface of the eye opposite the lens and includes the retina, optic disc (or the optic nerve head), macula, fovea and posterior pole. The arteries and veins enter the eye fundus through the optic disc. During the examination of the eye fundus, an examiner looks for the venous pulsation of the retinal veins. Venous pulsation provides information regarding, among other things, the intracranial pressure of the subject including indications of instances in which the intracranial pressure of the subject is elevated.

Venous pulsation is not spontaneous, however, for all subjects. As such, venous pulsation must sometimes be induced. In this regard, the examiner may apply pressure alongside the eye, such as by applying pressure through an eyelid, in order to induce the venous pulsation. There may be many variations, however, with respect to the application of the pressure utilized to induce venous pulsation including differences in the amount of pressure that is applied by different examiners and differences in the amount of pressure that is applied to different subjects by a single examiner. As the venous pulsation is at least partially dependent upon the amount of pressure that is applied, the variations associated with the amount of pressure that is applied in order to induce venous pulsation may make it more difficult to drawing conclusions based upon the venous pulsation and/or to compare the venous pulsation induced by the application of pressure during different eye examinations.

### BRIEF SUMMARY

Example methods and apparatus are provided in order to controllably induce venous pulsation, such as during an eye examination. In accordance with an example embodiment, the location at which the pressure is to be applied is defined in a consistent manner and the amount of pressure that is applied to the location proximate the eye of a subject is measured and may be associated with an image that is captured of the ensuing venous pulsation. Further, the timing of the application of the pressure to a location proximate the eye relative to the commencement of venous pulsation may be identified. Thus, the context associated with the venous pulsation may be more fully defined in accordance with an example embodiment, thereby facilitating the eye examination of the subject and more meaningful comparisons of the eye examination to other eye examinations of the same or different subjects.

In an example embodiment, an apparatus is provided that includes first means configured to sense an amount of pressure that is applied and transfer substantially the amount of pressure that is sensed to a subject undergoing examination. The first means is configured to transfer substantially the amount of pressure that is sensed to the subject to a location proximate an eye of the subject without making direct contact with a surface of the eye. The apparatus also includes second means, which may be the first means, configured to transmit a signal indicative of substantially the amount of pressure that is sensed (e.g., that is being transferred to the location proximate the eye of the subject) to a receiving device.

In an example embodiment, the first means is responsive to tactile input. The first means of an example embodiment includes a sensor that is flexible so as to conform to a contour of the location proximate the eye of the subject. The first means of another example embodiment includes an inflatable cuff positioned on an eyelid of the subject and configured to apply the pressure upon inflation. The apparatus of an example embodiment also includes guide means configured to provide an indicator associated with the location proximate the eye of the subject in order to guide application of the pressure to the location proximate the eye of the subject.

The apparatus of an example embodiment also includes positioning means for carrying and positioning the first means in alignment with the location proximate the eye of the subject. The positioning means of one example embodiment includes goggles configured to be worn by the subject such that the first means is positioned in alignment with an eyelid of a subject at a location spaced apart from the pupil of the eye. The apparatus of an example embodiment also includes image recording means configured to capture at least one image of at least part of a fundus of the eye including venous pulsation within the eye following receipt of the signal indicative of the amount of pressure that is sensed and that is being transferred to the subject. The image recording means of an example embodiment is further configured to capture a video of at least part of the fundus of the eye, some part of the video may include the venous pulsation within the eye depending on the condition of the subject, in response to receipt of the signal indicative of substantially the amount of pressure that is sensed and that is being transferred to the subject.

In another example embodiment, a method is provided that includes receiving a signal indicative of a transfer of substantially an amount of pressure applied to a location proximate an eye of the subject undergoing examination without making direct contact with a surface of the eye. The signal provides an indication of the amount of pressure applied to the location proximate the eye. The method also includes capturing at least one image of at least part of a fundus of the eye including venous pulsation within the eye following receipt of the signal indicative of the transfer of substantially the amount of pressure applied to the location proximate the eye.

The method of an example embodiment captures the at least one image of at least part of the fundus of the eye by capturing a video of at least part of the fundus of the eye, which may include the venous pulsation within the eye. The method of an example embodiment captures the at least one image of at least part of the fundus of the eye by capturing the at least one image of at least part of the fundus of the eye while pressure is applied to the location proximate the eye. The method of an example embodiment also includes identifying a time at which substantially the amount of pressure that was applied to the subject was transferred to the location proximate the eye relative to the at least one image of at least part of the fundus of the eye that is captured. The method of an example embodiment also includes causing an instruction regarding application of the amount of pressure to the location proximate the eye to be transmitted from a location remote from the subject. The method of an example embodiment also includes associating the at least one image of at least part of the fundus of the eye, which may include the venous pulsation within the eye, that is captured with the amount of pressure applied to the location proximate the eye, as indicated by the signal.

### BRIEF DESCRIPTION OF THE DRAWINGS

Having thus described certain example embodiments of the present disclosure in general terms, reference will hereinafter be made to the accompanying drawings, which are not necessarily drawn to scale, and wherein:
FIG. 1 is a perspective view of a subject having sensors positioned proximate their eyes and an ophthalmoscope including image recording means in accordance with an example embodiment of the present disclosure;
FIG.2 is a more detailed view of a single eye of the subject illustrating the sensor positioned proximate the eye of the subject in accordance with an example embodiment of the present disclosure;
FIG. 3 is a view of a subject having a sensor positioned on an inner portion of their eyelid in accordance with an example embodiment of the present disclosure;
FIG.4 is a side cross-sectional view of an inflatable cuff for applying pressure in accordance with an example embodiment of the present disclosure;
FIGs.5A and 5B are front and side views, respectively, of a subject wearing a pair of goggles for positioning the first means in alignment with an eyelid of the subject at a location spaced apart from the pupil of the eye in accordance with an example embodiment of the present disclosure;
FIG. 6 is a front view of a subject wearing an eye patch for positioning the first means in alignment with an eyelid of the subject at a location spaced apart from the pupil of one of their eyes in accordance with an example embodiment of the present disclosure;
FIG.7 is a block diagram of a system in accordance with an example embodiment of the present disclosure; and
FIG.8 is a flowchart illustrating operations performed, such as by the system of FIG. 7, in accordance with an example embodiment of the present disclosure.

### DETAILED DESCRIPTION

Some embodiments of the present invention will now be described more fully hereinafter with reference to the accompanying drawings, in which some, but not all, embodiments of the invention are shown. Indeed, various embodiments of the invention may be embodied in many different forms and should not be construed as limited to the embodiments set forth herein; rather, these embodiments are provided so that this disclosure will satisfy applicable legal requirements. Like reference numerals refer to like elements throughout. As used herein, the terms "data," "content," "information," and similar terms may be used interchangeably to refer to data capable of being transmitted, received and/or stored in accordance with embodiments of the present invention. Thus, use of any such terms should not be taken to limit the spirit and scope of embodiments of the present invention.

Additionally, as used herein, the term 'circuitry' may refer to one or more or all of the following: (a) hardware-only circuit implementations (such as implementations in analog circuitry and/or digital circuitry); (b) combinations of circuits and software, such as (as applicable): (i) a combination of analog and/or digital hardware circuit(s) with software/firmware and (ii) any portions of hardware processor(s) with software (including digital signal processor(s)), software, and memory(ies) that work together to cause an apparatus, such as a mobile phone or server, to perform various functions) and (c) hardware circuit(s) and/or processor(s), such as a microprocessor(s) or a portion of a microprocessor(s), that requires software (e.g., firmware) for operation, but the software may not be present when needed for operation. This definition of 'circuitry' applies to all uses of this term in this application, including in any claims. As a further example, as used in this application, the term 'circuitry' also covers an implementation of merely a hardware circuit or processor (or multiple processors) or portions of a hardware circuit or processor and its (or their) accompanying software and/or firmware. The term 'circuitry' also covers, for example and if applicable to the particular claim element, a baseband integrated circuit or processor integrated circuit for a mobile phone or a similar integrated circuit in a server, a cellular network device or other computing or network device.

As defined herein, a "computer-readable storage medium," which refers to a physical storage medium (e.g., volatile or non-volatile memory device), may be differentiated from a "computer-readable transmission medium," which refers to an electromagnetic signal.

A method and apparatus are provided in order to facilitate the application of pressure to a location proximate an eye of a subject in order to induce eye fundus retinal venous pulsation. An image of the eye, such as an image of at least part of the fundus of the eye, may include the venous pulsation, depending on the condition of the subject, may be captured and, in one embodiment, the amount of pressure applied to the location proximate the eye may be associated with the image of the eye. As such, the context associated with the inducement of venous pulsation may be recorded and considered in conjunction with the eye examination.

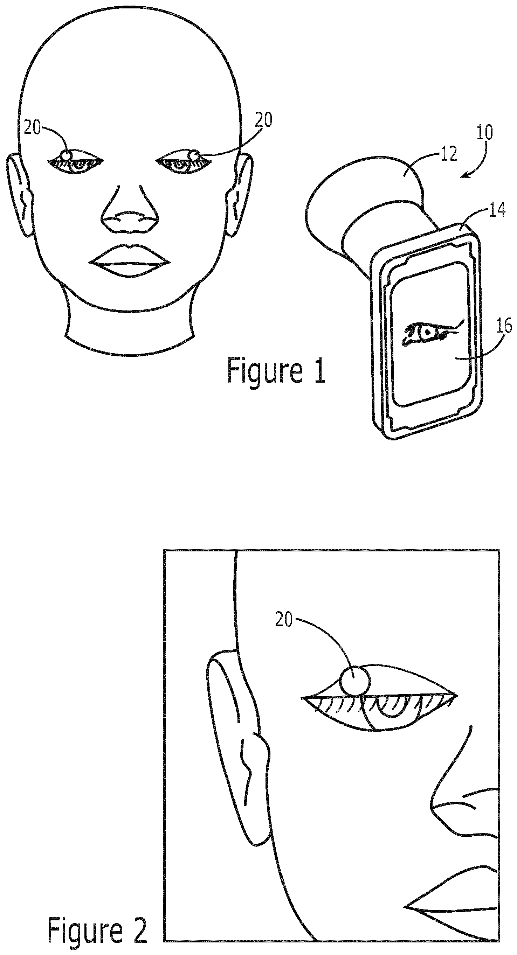

A subject undergoing an eye examination including an inspection of the eye fundus is depicted in FIG. 1. Although the subject is depicted to be a person, the subject may, instead, be an animal. In this regard, an ophthalmoscope 10 is depicted to permit the examiner, such as a doctor, optometrist, physician assistant trained nurse or the like, to examine the eye of the subject. Although the ophthalmoscope may be configured in various manners without departing from the spirit and scope of the invention, the ophthalmoscope of the illustrated embodiment includes an eye piece 12, typically formed of a pliable material, such as rubber. The eye piece of the illustrated embodiment has a frustoconical shape with a distal end that is configured to interface with the subject defining a larger interior area than the proximal end that connects to the remainder of the ophthalmoscope. The ophthalmoscope may be positioned relative to the eye of a subject such that the interface surrounds the eye. The ophthalmoscope of this example embodiment also includes image recording means 14 configured to capture at least one image of the eye, such as at least one image of at least part of the fundus of the eye. In an example embodiment, the image recording means is configured to capture a video of the eye, such as a video of at least part of the fundus of the eye. The image recording means may be embodied by a camera or other image sensor configured to capture an image, such as a video, of the eye. In the illustrated embodiment, the ophthalmoscope not only includes a camera configured to capture a video of the eye, but also a display 16 configured to present the image of the eye that is captured by the camera, such as for real-time review by the examiner. The ophthalmoscope of an example embodiment also includes or is otherwise in communication or association with, a memory device for storing the at least one image, such as a video, captured of the eye.

During the eye examination, the examiner studies the eye, including the fundus and captures an image of the optical fundus, such as with the camera of the ophthalmoscope 10. During the examination of the optical fundus, the examiner generally considers the venous pulsation of the retinal veins which, in turn, provides information regarding the health of the subject including, for example, the intracranial pressure, such as may be elevated in response to brain trauma, a stroke or a brain tumor, certain eye diseases, such as glaucoma, that cause changes in venous pulsation and/or different vascular diseases. In instances in which the venous pulsation is not spontaneous, the examiner may in some cases induce the venous pulsation or at least attempt to induce the venous pulsation even though the venous pulsation continues to be undetectable even after pressure is induced on the eyelid.

To facilitate the venous pulsation, an apparatus of an example embodiment includes first means configured to sense an amount of pressure and to transfer the same amount of pressure or substantially the same amount of pressure applied to the subject. As used herein, "substantially the same amount of pressure" means approximately the same amount of pressure. For example, the difference between the amount of pressure sensed and the amount of pressure transferred by the first means is within an acceptable or predefined tolerance. In this regard, the first means is configured to transfer substantially the same amount of pressure that is applied to a location proximate an eye of the subject to the subject and, more particularly, to the eye of the subject without making direct contact with the actual surface of the eye. The first means of an example embodiment transfers the same amount of pressure that is sensed to the subject, such as to the location proximate the eye of the subject and, in turn, to the eye itself. However, in some embodiments, a portion of the pressure sensed by the first means is not transferred to the subject and, instead, serves to, for example, deform the first means or otherwise be redirected. However, the majority of the pressure applied to the location proximate the eye is transferred by the first means to the subject, such as the eye of the subject. As such, the transfer of substantially the amount of pressure applied to and sensed by the first means to the subject refers to the transfer, in one embodiment, of at least 90% of the pressure applied to the location proximate the eye of the subject to the subject and, in other embodiments at least 95%, 98% or 99% of the pressure applied to the location proximate the eye of the subject to the subject.

The first means may be embodied in various manners. In one example embodiment, the first means includes a sensor 20 that is flexible so as to conform to a contour of the location proximate the eye of the subject. As shown in FIG. 1 and, in more detail in FIG. 2, the sensor of an example embodiment is a flexible film, such as formed of a plastic or other pliable material. The sensor is positioned at the location proximate the eye of the subject to which the pressure applied to the sensor is to be, in turn, applied to the eye of the subject. In this regard, the sensor is responsive to tactile input which serves to apply the pressure. Thus, the person conducting the eye examination may apply tactile input to the sensor, such as by contacting the sensor with their finger and applying the pressure, that is sensed by the sensor and is substantially transferred to the eye of the subject in order to cause the venous pulsation to be induced.

The sensor 20 of an example embodiment includes a pressure sensor configured to sense the amount of pressure applied to the sensor. The pressure sensor may be embodied in any of a wide variety of different manners. In one embodiment, however, the sensor includes resonant circuitry, such as described, by way of example but not of limitation, Chen, et al., "Continuous wireless pressure monitoring and mapping with ultra-small passive sensors for health monitoring and critical care", Nature Communications (October 6, 2014), configured to generate an output signal, such as a wireless signal, e.g., a radio frequency signal, having a characteristic, such as a frequency or wavelength, that is responsive to and dependent upon and that controllably varies in a predefined manner based upon the pressure applied to the sensor. Thus, as different amounts of pressure are applied to the sensor of this example embodiment, the characteristic of the output signal correspondingly varies in a predefined manner. Thus, analysis of the signals provided by the resonant circuitry may provide information regarding the amount of pressure applied to the location proximate the eye of the subject.

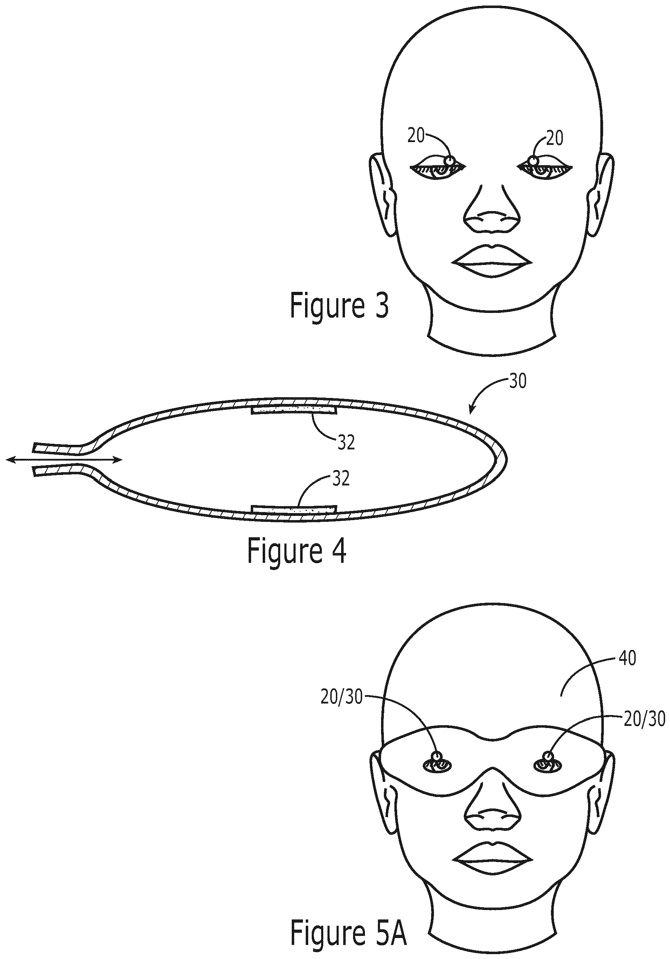

The first means may be embodied in other manners. For example, the first means of another example embodiment may be embodied by an inflatable cuff 30, such as shown in FIG. 4. The inflatable cuff is positioned at the location proximate the eye of the subject without making direct contact with the surface of the eye. For example, the inflatable cuff may be positioned on an eyelid of the subject, such as at the location at which the sensor is shown in FIGs. 1 and 2. The inflatable cuff is configured to be alternately inflated and deflated. By holding the inflatable cuff in position at the location proximate the eye of the subject, the inflation of the inflatable cuff is configured to apply pressure, such as to the location proximate the eye of the subject. Conversely, when deflated, the inflatable cuff removes the pressure or no longer applies pressure, such as to the location proximate the eye of the subject. One example of an inflatable cuff is shown in FIG. 4. The inflatable cuff is formed of a pliable material, such as a plastic or the like, that is configured to be alternately inflated and deflated. The inflatable cuff also includes a pressure sensor. As schematically represented by the capacitor plates 32 carried by the opposed surfaces of the inflatable cuff, the inflatable cuff of an example embodiment may include resonant circuitry, such as described above in conjunction with the sensor and one example embodiment of which is described in the Chen, et al. article entitled "Continuous wireless pressure monitoring and mapping with ultra-small passive sensors for health monitoring and critical care". In this example embodiment, pressure limits may be established relative to the amount of pressure applied by the inflatable cuff, such as by limiting the pressure to which the inflatable cuff is inflated to be no greater than a predefined threshold, thereby insuring the subject's safety during the eye examination.

Regardless of the manner in which the firm means is embodied, the first means may be positioned at various locations proximate the eye of the subject, such as within the eye socket, so long as the pressure applied via the first means is transferred to the eye of the subject without directly contacting the surface of the eye. As such, the sensor 20 (or alternatively the inflatable cuff 30) may be positioned, as shown in FIGs. 1-3, on an eyelid of the subject, such as on that portion of the eyelid that is laterally disposed, either to the inside as shown in FIG. 3 or to the outside as shown in FIGs. 1 and 2, with respect to the portion of the eye that is visible. As such, pressure applied to the sensor (or alternatively the inflatable cuff 30) of FIGs. 1 and 2 is transferred to the eye itself in order to induce venous pulsation. As noted, the first means, such as the sensor or the inflatable cuff, may be positioned at other locations proximate the eye of the subject, such as on the exterior surface of the upper eyelid, on the exterior surface of the lower eyelid or on the interior surface of either the upper eyelid or the lower eyelid between the respective eyelid and the surface of the eye.

Since the first means, such as the sensor 20 or the inflatable cuff 30, is positioned at a location proximate the eye of the subject without making direct contact with the surface of the eye, the fundus of the eye is able to be viewed through the pupil, which is beneficial during the eye examination during which the examiner studies the eye including the eye fundus and, as a result, desirably has a view through the pupil to the underlying eye fundus. Further, the positioning of the first means, such as the sensor or the inflatable cuff, at a location proximate the eye without making direct contact with the surface of the eye may facilitate its use, particularly by subjects who are reluctant to place objects upon or otherwise touch the surface of their eye and for the healthcare professional who may otherwise require specialized training to place a lens or other object on the eye surface of a subject. Further, the positioning of the first means, such as the sensor or the inflatable cuff, at a location proximate the eye without making direct contact with the surface of the eye reduces the likelihood that any contaminates potentially carried by the first means will be introduced into the eye of a subject, thereby maintaining the sterility of the eye. Further, the positioning of the first means, such as the sensor or the inflatable cuff, at a location proximate the eye of the subject without making direct contact with the surface of the eye avoids the application of force directly on the eye which could deleteriously change the shape of the eyeball which may, in turn, create optical distortions during the eye examination and also avoids a source of potential damage to the surface of the eye that could otherwise result in pressure damage, scarring, pain and/or a risk of infection.

In an alternative embodiment, the sensor 20 is disposed, not at the location proximate the eye of the subject as shown in FIGs. 1 and 2, but on the fingertip of the person conducting the eye examination. In this example embodiment, the fingertip may be placed at the location proximate the eye of the subject, thereby bringing the sensor into position at the location proximate the eye of the patent. Pressure may then be applied to the location and, in turn, to the eye of the subject without making direct contact with the surface of the eye with the sensor carried by the fingertip sensing the pressure that is applied by the finger. For example, the fingertip may be placed at the same location on the eyelid of the subject as the location at which the sensor is depicted in FIGs. 1 and 2, such as on the upper or lower eyelid of the subject and, in an example embodiment, on the upper or lower eyelid near or toward the corner of the eye.

The apparatus of an example embodiment also includes guide means configured to provide an indicator, such as a visible indicator, associated with the location proximate the eye of the subject in order to guide the application of the pressure to the location proximate the eye of the subject. For example, the visible indicator may advise the person conducting the eye examination as to the location at which to place the first means, such as the sensor 20 or the inflatable cuff 30. Alternatively, in the case in which the sensor is already in position at the location proximate the eye of the subject, the visible indicator may advise the person conducting the eye examination of the location at which pressure should be applied, such as the location that the person conducting the eye examination should place their finger and apply pressure. The visible indicator may be provided in various manners. In one example embodiment, the visible indicator is provided by color indicia that indicates the location at which pressure is to be applied. For example, the sensor, such as that shown in the embodiment of FIGs. 1 and 2, may have a distinctive color, such as a red color, in order to guide the application of pressure to the location having the visual indicator.

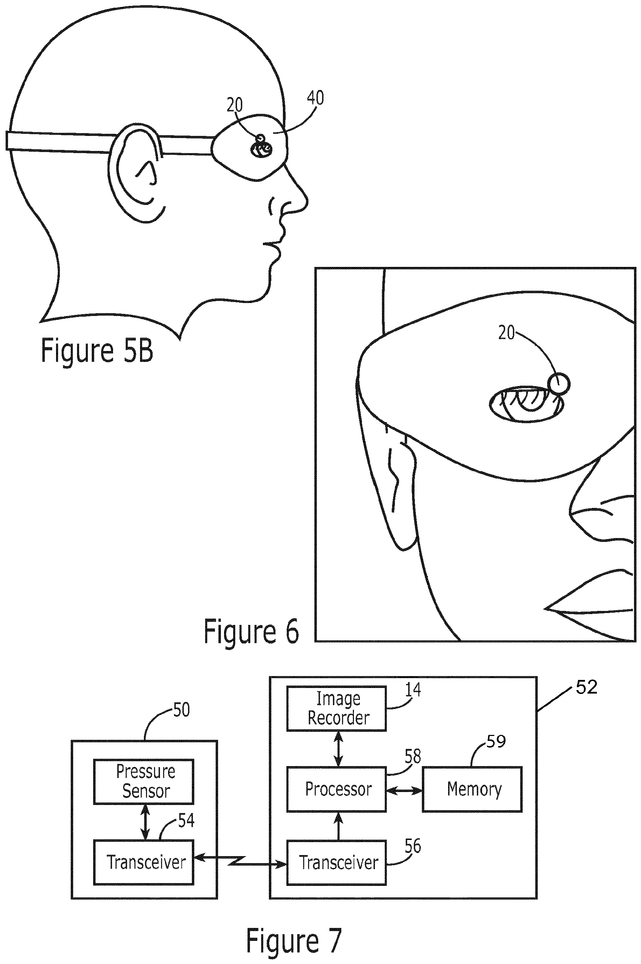

The apparatus of an example embodiment also includes positioning means for carrying and positioning the first means in alignment with the location proximate the eye of the subject. Although the positioning means may be embodied in various manners, the positioning means of one example embodiment includes goggles 40 configured to be worn by the subject such that the first means is positioned in alignment with the location proximate the eye of the subject. For example, the goggles may be configured such that the first means carried by the goggles is positioned in alignment with an eyelid of the subject at a location spaced apart from the pupil of the eye once the subject has donned the goggles. In this regard, the pupil may serve as a landmark from which the distance to the location at which pressure is applied is calculated. As shown in FIGs. 5A and 5B, the pair of goggles of one example embodiment fits over the eyes of the subject and conforms to that portion of the face of the subject surrounding their eyes. As such, the goggles may be formed of a flexible, resilient material, such as rubber, and, in some embodiments are formed of dispensable and washable material to permit repeated use of the goggles. The goggles define openings through which the eyes of the subject are visible, thereby permitting the eye examination to be conducted in an unimpeded manner. The goggles also carry the first means, such as the sensor 20 or inflatable cuff 40, at a location relative to the openings defined for the eyes of the subject such that the first means is in alignment with the location proximate the eye of the subject. As shown in the embodiment of FIGs. 5A and 5B, the first means is positioned in alignment with an eyelid of the subject, such as that portion of the eyelid on the outside of the eye of the subject, at a location spaced from the pupil of the eye.

The goggles 40 of an example embodiment may also include guide means. For example, the goggles and/or the first means carried by the goggles may provide a visible indicator, such as color indicia, that guides application of the pressure to the first means at the location proximate the eye of the subject. Further, while the goggles may include first means positioned at a location proximate one eye of the subject, the goggles may include first means positioned at first and second locations proximate the left and right eyes, respectively, of the subject, such as shown in FIG. 5A. As such, a subject may don the goggles prior to the eye examination and may subsequently remove the goggles once the eye examination has concluded or at least once the examination of the eye fundus including the venous pulsation has been completed. In an embodiment in which the goggles include first means positioned at first and second locations proximate the left and right eyes, respectively, of the subject, both eyes may be examined, including an examination of the venous pulsation of both eyes, while wearing the goggles. In another example embodiment, the positioning means for carrying and positioning the first means in alignment with the location proximate the eye of the subject is configured to position the first means in alignment with the location proximate one, but not both eyes of the subject. As shown in FIG. 6, for example, the positioning means may be an eyepatch having an opening therein through which the subject can see. The eyepatch of this example carries a sensor in alignment with the upper eyelid of the subject to sense pressure applied through the upper eyelid to the eye of the user.

In an example embodiment as shown in FIG. 7, a system includes a subject module 50 and an image capture module 52 in communication with one another. As shown in FIG. 7, the subject module 50 is a hardware module that includes circuitry and the image capture module 52 is a hardware module that includes circuitry. The subject module 50 and an image capture module 52 may be configured to communicate wirelessly with one another. In other embodiments, the subject module and the image capture module are configured to communicate in other manners, such as via wireline connection. In an example embodiment, the apparatus also includes second means, which may be the same as the first means, configured to transmit a signal indicative of substantially the amount of pressure that is sensed (e.g., the amount of pressure intended to transfer to the subject) to a receiving device. A receiving device may be any device, sensor, or apparatus that is implemented to use the signal or information indicative of substantially the amount of pressure that is sensed. For example, a receiving device (not shown) may display the amount of pressure to show the amount of pressure being sensed, which is transferred, or intended to be transferred, to the subject). Another example receiving device may be an image capture module 52.

In the illustrated embodiment, the subject module 50 includes the second means embodied as a transceiver 54 configured to transmit signals to and receive signals from the image capture module 52. Alternatively, the second means may be embodied by a transmitter for transmitting signals to the image capture module. As shown in FIG. 7, the image capture module of one example embodiment also includes a transceiver 56 configured to receive signals from and transmit signals to the subject module. Alternatively, the image capture module may include a receiver for receiving signals from the subject module.

In example embodiment described above in which the first means includes a pressure sensor embodied by, for example, resonant circuitry, the resonant circuitry may be configured to not only sense the amount of pressure that is applied, but also to serve as the transceiver 54 or transmitter so as to transmit a signal having a characteristic, such as wavelength or frequency, indicative of substantially the amount of pressure that is applied to the subject and that is sensed by the first means and transferred to the subject. In an example embodiment, the resonant circuitry may passive and, as a result, may be responsive to an interrogation signal, such as provided by the image capture module 52 and, more particularly, by the transceiver 56 or transmitter of the image capture module. In this embodiment, the resonant circuitry is configured to transmit a signal in response to the interrogation signal in which a characteristic of the signal, such as the frequency or wavelength of the signal, is indicative of the amount of pressure sensed by the first means and substantially applied to the eye of the subject. As a result of its passive configuration, the resonant circuitry of this example embodiment utilizes energy from the interrogation signal in order to provide the energy necessary to transmit the signal that provides information to the image capture module regarding the amount of pressure applied to the eye of the subject. In an alternative embodiment, the second means include an active transceiver or transmitter that draws power from a battery or other source of power for transmitting the signal providing information regarding the amount of pressure applied to the eye of the subject. As also shown in FIG. 7 and discussed above, the signal that is transmitted by the subject module 50 indicative of substantially the amount of pressure that is applied to the subject and that is being transferred to the eye of the subject is received, such as by a transceiver 56 or receiver of the image capture module 52.

In the illustrated embodiment, an image capture module 52 includes not only communication means, such as the transceiver 56 or receiver, for receiving the signal indicative of substantially the amount of pressure being applied to the eye of the subject from the subject module 50, but also the image recording means 14, such as a camera or video recorder. As such, the image capture module of one embodiment may be embodied by an ophthalmoscope 10, such as shown in FIG. 1. In an example embodiment, the image capture module also includes processing means, such as processing circuitry, e.g., a processor 58, configured to process and/or facilitate storage of the image(s) captured by the image recording means and the signals received from the subject module. The processor may be embodied in a number of different ways. For example, the processor may be embodied as one or more of various hardware processing means such as a coprocessor, a microprocessor, a controller, a digital signal processor (DSP), a processing element with or without an accompanying DSP, or various other circuitry including integrated circuits such as, for example, an ASIC (application specific integrated circuit), an FPGA (field programmable gate array), a microcontroller unit (MCU), a hardware accelerator, a special-purpose computer chip, or the like. As such, in some embodiments, the processor may include one or more processing cores configured to perform independently. A multicore processor may enable multiprocessing within a single physical package. Additionally or alternatively, the processor may include one or more processors configured in tandem via the bus to enable independent execution of instructions, pipelining and/or multithreading.

In an example embodiment, the processor 58 may be configured to execute instructions stored in the memory device 59 or otherwise accessible to the processor. Alternatively or additionally, the processor may be configured to execute hard coded functionality. As such, whether configured by hardware or software methods, or by a combination thereof, the processor may represent an entity (e.g., physically embodied in circuitry) capable of performing operations according to an embodiment of the present disclosure while configured accordingly. Thus, for example, when the processor is embodied as an ASIC, FPGA or the like, the processor may be specifically configured hardware for conducting the operations described herein. Alternatively, as another example, when the processor is embodied as an executor of instructions, the instructions may specifically configure the processor to perform the algorithms and/or operations described herein when the instructions are executed. However, in some cases, the processor may be a processor of a specific device (e.g., an image processing system) configured to employ an embodiment of the present invention by further configuration of the processor by instructions for performing the algorithms and/or operations described herein. The processor may include, among other things, a clock, an arithmetic logic unit (ALU) and logic gates configured to support operation of the processor.

In an example embodiment, the processing circuitry is configured to associate the amount of pressure applied to the location proximate the eye, as indicated by the signal from the subject module 50, with at least one image of the eye, which may include the venous pulsation within the eye, that is captured by the image recording means 14 during or immediately following the application of the pressure, such as the venous pulsation induced by the application of pressure proximate the eye. For example, the processing circuitry may direct the storage of the information regarding the amount of pressure applied to the location proximate the eye, as indicated by the signal, in memory 59 in association with the at least one image of the eye, such as at least one image of at least part of the fundus of the eye, which may include the venous pulsation, that is captured during or immediately following the application of the pressure.

The signal provided by the subject module 50 indicative of substantially the amount of pressure that is applied to the subject that is being transferred to the subject may also include timing information providing an indication as to the time at which the pressure was applied, such as the initial time at which the pressure was applied and the time duration for which the pressure was applied. The timing information may be provided in various manners, but in an embodiment in which a resonant circuitry provides a signal having a characteristic, such as wavelength or frequency, indicative of the pressure that has been applied, the timing information may be provided by the time at which the characteristic is altered from a nominal value in response to the application of force and the length of time that the characteristic remains in the altered state in response to the application of force. In this embodiment, the processing circuitry may also or alternatively be configured to associate the timing information with at least one image of the eye, which may include the venous pulsation within the eye, that is captured by the image recording means during or immediately following the application of the pressure, such as the venous pulsation induced by the application of pressure proximate the eye. For example, the processing circuitry may direct the storage of the timing information, as indicated by the signal, in memory 59 in association with the at least one image of the eye including the venous pulsation that is captured during or immediately following the application of the pressure.

In an embodiment in which the second means, such as the transceiver 54 or transmitter, of the subject module 50 is passive, the image capture module 52, such as the communication means, e.g., the transceiver 56, may also be configured to transmit interrogation signals to the subject module, such as to resonant circuitry in order to illicit the transmission of a signal indicative of substantially the amount of pressure that is applied to the subject that is being transferred to the subject. The interrogation signals may be transmitted continuously throughout the eye examination, at regular intervals during the eye examination or in any other sequence or pattern that is desired.

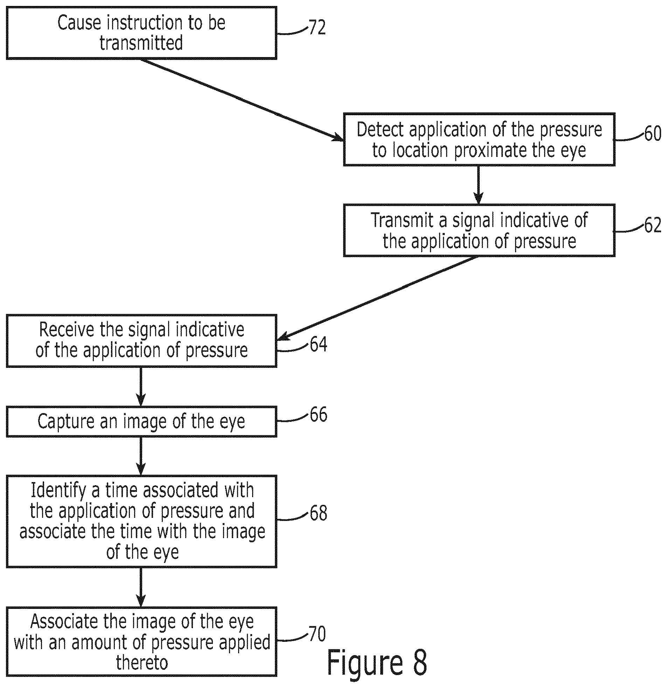

During operation and as shown in FIG. 8 in which blocks in the left hand column are generally performed by the image capture module 52, such as may be embodied by an ophthalmoscope 10, and blocks in the right hand column are generally performed by the subject module 50, the subject module, such as the first means, e.g., the sensor 20 or the inflatable cuff 30, is configured to detect application of pressure to a location proximate the eye without making direct contact with the surface of the eye. See block 60. As noted above, the application of pressure to a location proximate the eye of the subject, such as to a location on the eye lid of the subject, may be guided, such as by guide means that provides a visible indicator of the location to which the pressure should be applied. In some example embodiments, auditory and/or tactile indicators may also be provided to guide the application of pressure to a location proximate the eye of the subject. For example, the subject module or the image capture module may generate and output a first sound, such as a first chime or a first beep, indicating that pressure should be applied and/or a second sound, such as a second chime or a second beam, indicating that pressure being applied should be released. Additional sounds may be generated and output indicative of the application of different levels of pressure in order to guide the eye examination. Additionally or alternatively, the subject module or the image capture module may generate a tactile output, such as a vibration indicating that pressure should be applied, indicating that that pressure being applied should be released and/or indicating that different levels of pressure should be applied in order to similarly guide the eye examination. The subject module, such as the second means, e.g., the transceiver 54 or transmitter, is then configured to transmit a signal indicative of the transfer of substantially the amount of pressure applied to a location proximate the eye of the subject without making direct contact with the surface of the eye, such as a signal having a characteristic that is indicative of the amount of pressure that is applied to the location proximate the eye. See block 62.

As shown in block 64, the image capture module 52, such as the communications means, e.g., the transceiver 56 or receiver, is configured to receive the signal indicative of the transfer of substantially the amount of pressure applied to a location proximate the eye of the subject without making direct contact with the surface of the eye. The signal provides an indication of the amount of pressure applied to the location proximate the eye. In an example embodiment, the signal also provides timing information indicative of the time at which the pressure is applied to the eye, such as the initial time at which the pressure is first applied as well as the duration, that is, the length of time for which the pressure is applied. The image capture module, such as the image recording means 14, e.g., a camera, is also configured to capture at least one image of the eye, such as at least one image of at least part of the fundus of the eye, including the venous pulsation within the eye. See block 66. In an example embodiment, the at least one image of the eye is captured following receipt of the signal indicative of the transfer of substantially amount of pressure applied to the location proximate the eye. In other words, the at least one image of the eye is triggered by the signal such that the at least one image of the eye is captured following the application of pressure to the eye and the inducement of venous pulsation therein. In some embodiments, an image of the eye may be captured prior to the application of pressure with the capture of an image of the eye being continued during and following the application of pressure to the eye in order to capture images during the time at which the venous pulsation commences and throughout the duration of the venous pulsation.

As shown in block 68, the image capture module 52, such as the processing means, e.g., the processing circuitry, may be configured to identify timing information associated with the application of pressure to a location proximate the eye from the signal provided by the subject module 50 and may associate the timing information with an image of the eye that includes the venous pulsation induced by the application of pressure, thereby effectively time stamping the image of the eye. In this regard, the processing circuitry may cause both the timing information and the corresponding image of the eye to be stored in memory 59 in association with one another. Additionally or alternatively, as shown in block 70, the image capture module, such as the processing means, e.g., the processing circuitry, may be configured to associate the amount of pressure applied to a location proximate the eye as indicated by the signal provided by the subject module with an image of the eye, such as an image of at least part of the fundus of the eye, that includes the venous pulsation induced by the application of pressure. In this regard, the processing circuitry may cause both the information regarding the amount of pressure applied to a location proximate the eye and the corresponding image of the eye to be stored in memory in association with one another. As such, an examiner may obtain a greater appreciation for the context of the eye examination during the inducement of the venous pulsation including the amount of pressure that was applied and the time at which the pressure was applied relative to the resulting venous pulsation. Thus, the examiner may be better able to interpret the results of the eye examination and to compare the results of different examinations.

In some embodiments, the pressure is not simply applied in order to induce venous pulsation, but, instead, pressure is applied to induce venous pulsation at a first pressure level and the pressure that is applied is then gradually increased from the first pressure level following the initial detection of venous pulsation. The increase in applied pressure may cause the venous pulse to increase, such as by increasing in pulse rate and/or amplitude, until a second pressure level at which further increases in the applied pressure does not result in any further increase in the venous pulse. Subsequently, the pressure may be gradually decreased until a third pressure level is reached at which the venous pulsation is no longer detected. The third pressure level may differ from the first pressure level. The first, second and third pressure levels may be detected, such as by the subject module 50, such as the first means, e.g., the sensor 20 or the inflatable cuff 30, and the times at which venous pulse is initially induced, ceases to increase and then is no longer detected may also be identified, such as by the image capture module 52, such as the processing means, e.g., the processing circuitry, and associated with the first, second and third pressure levels, respectively. Based upon the first, second and third pressure levels, a pressure curve including the first, second and third pressure levels may be defined and the pressure curve may be associated with images, such as a video recording, of the eye fundus that are captured at the times associated with the first, second and third pressure levels. For example, the pressure curve including the first, second and third pressure levels may be associated with respective images of a video recording captured at least from the time at which the first pressure level induces venous pulsation to the time at which the venous pulsation ceases at the third pressure level.

By defining a pressure curve, the pressure curves associated with the same subject at different points in their life may be compared in order to derive information regarding changes in the subject's eye. Additionally or alternatively, the pressure curve associated with a respective subject may be compared to the pressure curve(s) associated with one or more other subjects in order to derive comparative information with respect to the respective subject.

While an examiner may conduct the eye examination in person, the eye examination may be conducted remotely, such as in a telemedicine application. In this example embodiment, a subject may be directed to look into an ophthalmoscope 10, such that a camera of the ophthalmoscope captures an image of the eye including the optical fundus of the subject. In this example embodiment, a healthcare professional, such as an ophthalmologist, neurologist or other health care professional, all of which are termed an examiner herein, located remote from the subject may issue an instruction directing the subject to apply pressure at a location proximate the eye of the subject without making direct contact with the surface of the eye. For example, the instructions may direct the subject to apply pressure at a location designated by the guide means. As shown in block 72 of FIG. 8, the image capture module 52, such as the communication means, e.g., the transceiver 56, may be configured to transmit an instruction to the subject, such as via the second means, e.g., the transceiver 54, of the subject module 50. A signal may then be generated indicative of the amount of pressure that has been applied and the time at which the pressure was applied and an image of the eye including the venous pulsation induced by the application of pressure may be captured and provided by the subject module to the image capture module. The healthcare professional may then review the images and the related information including, for example, the amount of pressure applied to induce the venous pulsation, in order to provide feedback to the subject regarding the condition of their eyes, even though the subject and the healthcare professional are located remote from one another.

As described above, FIG. 8 is a flowchart of an apparatus, method, and computer program product according to certain example embodiments. It will be understood that each block of the flowchart, and combinations of blocks in the flowchart, may be implemented by various means, such as hardware, firmware, processor, circuitry, and/or other devices associated with execution of software including one or more computer program instructions. For example, one or more of the procedures described above may be embodied by computer program instructions. In this regard, the computer program instructions which embody the procedures described above may be stored by a memory device of an apparatus employing an embodiment of the present invention and executed by processing circuitry or a processor of the apparatus. As will be appreciated, any such computer program instructions may be loaded onto a computer or other programmable apparatus (e.g., hardware) to produce a machine, such that the resulting computer or other programmable apparatus implements the functions specified in the flowchart blocks. These computer program instructions may also be stored in a computer-readable memory that may direct a computer or other programmable apparatus to function in a particular manner, such that the instructions stored in the computer-readable memory produce an article of manufacture, the execution of which implements the function specified in the flowchart blocks. The computer program instructions may also be loaded onto a computer or other programmable apparatus to cause a series of operations to be performed on the computer or other programmable apparatus to produce a computer-implemented process such that the instructions which execute on the computer or other programmable apparatus provide operations for implementing the functions specified in the flowchart blocks.

A computer program product is therefore defined in those instances in which the computer program instructions, such as computer-readable program code portions, are stored by at least one non-transitory computer-readable storage medium with the computer program instructions, such as the computer-readable program code portions, being configured, upon execution, to perform the functions described above, such as in conjunction with the flowchart of FIG. 6. In other embodiments, the computer program instructions, such as the computer-readable program code portions, need not be stored or otherwise embodied by a non-transitory computer-readable storage medium, but may, instead, be embodied by a transitory medium with the computer program instructions, such as the computer-readable program code portions, still being configured, upon execution, to perform the functions described above.

Accordingly, blocks of the flowchart support combinations of means for performing the specified functions and combinations of operations for performing the specified functions for performing the specified functions. It will also be understood that one or more blocks of the flowchart, and combinations of blocks in the flowchart, may be implemented by special purpose hardware-based computer systems which perform the specified functions, or combinations of special purpose hardware and computer instructions.

In relation to a computer program product, the operations performed by the image capture module 52 may be performed at the direction of computer program instructions. In this regard, computer program instructions could be configured to receive a signal from the subject module 50 providing an indication of the application of pressure and to also cause an image of the eye to be capture, such as by the image recording means 14. See blocks 64 and 66 of FIG. 6. Further, the computer program instructions of an example embodiment may be configured to associate timing information and/or information regarding the amount of pressure that was applied with the image of the eye that is captured, such as in conjunction with directing the storage of the image of the eye and the associated information in memory 59. See blocks 68 and 70. Further, in an embodiment in which the eye examination is to be conducted remotely, the computer program instructions may be configured to cause an instruction to be transmitted, such as to the subject module, regarding the manner in which pressure is to be applied to induce venous pulsation.

In relation to the operations performed by the subject module 50, the computer program instructions may be configured to receive a signal from the first means, such as the sensor 20 or inflatable cuff 30, that provides an indication as to the pressure applied to a location proximate the eye of the subject including the amount of pressure that was applied and, in some embodiments, timing information associated with the application of the pressure. See block 60 of FIG. 6. The computer program instructions of this example embodiment are also configured to cause the signal indicative of the application of pressure to the location proximate the eye of the subject to be transmitted, such as to the image capture module 52. See block 62.

In some embodiments, certain ones of the operations above may be modified or further amplified. Furthermore, in some embodiments, additional optional operations may be included. Modifications, additions, or amplifications to the operations above may be performed in any order and in any combination.

Many modifications and other embodiments of the inventions set forth herein will come to mind to one skilled in the art to which these inventions pertain having the benefit of the teachings presented in the foregoing descriptions and the associated drawings. Therefore, it is to be understood that the inventions are not to be limited to the specific embodiments disclosed and that modifications and other embodiments are intended to be included within the scope of the appended claims. Moreover, although the foregoing descriptions and the associated drawings describe example embodiments in the context of certain example combinations of elements and/or functions, it should be appreciated that different combinations of elements and/or functions may be provided by alternative embodiments without departing from the scope of the appended claims. In this regard, for example, different combinations of elements and/or functions than those explicitly described above are also contemplated as may be set forth in some of the appended claims. Although specific terms are employed herein, they are used in a generic and descriptive sense only and not for purposes of limitation.

## Claims

1. An apparatus comprising:
first means configured to sense an amount of pressure that is applied and transfer substantially the amount of pressure that is sensed to the subject undergoing examination, wherein the first means is configured to transfer substantially the amount of pressure that is sensed to the subject to a location proximate an eye of the subject without making direct contact with a surface of the eye; and
second means configured to transmit a signal indicative of substantially the amount of pressure that is sensed to receiving device.

2. An apparatus according to Claim 1 wherein the first means comprises a sensor that is flexible so as to conform to a contour of the location proximate the eye of the subject.

3. An apparatus according to Claim 1 wherein the first means comprises an inflatable cuff positioned on an eye lid of the patent and configured to apply the pressure upon inflation.

4. An apparatus according to any one of Claims 1 to 3 further comprising guide means configured to provide an indicator associated with the location proximate the eye of the subject in order to guide application of the pressure to the location proximate the eye of the subject.

5. An apparatus according to any one of Claims 1 to 4 further comprising positioning means for carrying and positioning the first means in alignment with the location proximate the eye of the subject.

6. An apparatus according to Claim 5 wherein the positioning means comprises goggles configured to be worn by the subject such that the first means is positioned in alignment with an eye lid of the subject at a location spaced apart from a pupil of the eye.

7. An apparatus according to any one of Claims 1 to 6 wherein the first means is responsive to tactile input.

8. An apparatus according to any one of Claims 1 to 7 further comprising image recording means configured to capture at least one image of at least part of a fundus of the eye, which may include venous pulsation within the eye, following receipt of the signal indicative of substantially the amount of pressure that is sensed.

9. An apparatus according to Claim 8 wherein the image recording means is further configured to capture a video of at least part of the fundus of the eye, which may include the venous pulsation within the eye, in response to receipt of the signal indicative of substantially the amount of pressure that is sensed.

10. A method comprising:
receiving a signal indicative of a transfer of substantially an amount of pressure applied to a location proximate an eye of a subject undergoing examination without making direct contact with a surface of the eye, wherein the signal provides an indication of the amount of pressure applied to the location proximate the eye; and
following receipt of the signal indicative of the transfer of substantially the amount of pressure applied to the location proximate the eye, capturing at least one image of at least part of a fundus of the eye, which may include venous pulsation within the eye.

11. A method according to Claim 10 wherein capturing the at least one image of at least part of the fundus of the eye comprises capturing a video of at least part of the fundus of the eye, which may include the venous pulsation within the eye.

12. A method according to any one of Claims 10 or 11 wherein capturing the at least one image of at least part of the fundus of the eye comprises capturing the at least one image of at least part of the fundus of the eye while pressure is applied to the location proximate the eye.

13. A method according to any one of Claims 10 to 12 further comprising identifying a time at which substantially the amount of pressure that was applied to the subject was transferred to the location proximate the eye relative to the at least one image of at least part of the fundus of the eye that is captured.

14. A method according to any one of Claims 10 to 13 further comprising causing an instruction regarding application of the amount of pressure to the location proximate the eye to be transmitted from a location remote from the subject.

15. A method according to any one of Claims 10 to 14 further comprising associating the at least one image of at least part of the fundus of the eye, which may include the venous pulsation within the eye, that is captured with the amount of pressure applied to the location proximate the eye, as indicated by the signal.
